# EUROPÄISCHE PATENTSCHRIFT

(11) **EP 1 477 203 B1**
(45) Veröffentlichungstag und Bekanntmachung des Hinweises auf die Patenterteilung: **02.09.2015**
(21) Anmeldenummer: 04090173.8
(22) Anmeldetag: 05.05.2004
(51) Int. Cl.: A61N 1/05

(54) **Epicard-Elektrode**
Epicardial electrode
Electrode épicardique

(30) Priorität: 15.05.2003 DE 10323016
(43) Veröffentlichungstag der Anmeldung: 17.11.2004
(73) Patentinhaber: BIOTRONIK SE & Co. KG, 12359 Berlin (DE)
(72) Erfinder: Flach, Erhard, 12305 Berlin (DE); Hahnke, Gerhard, 12623 Berlin (DE)
(74) Vertreter: Galander, Marcus

(56) Entgegenhaltungen:
- EP-A- 0 134 367
- US-A- 4 142 530
- US-A- 4 357 946
- US-A- 5 545 207

## Beschreibung

Die vorliegende Erfindung betrifft Einführbesteck zum minimalinvasiven Einführen einer Epikard-Elektrode in den Körper nach Anspruch 1. Die vorliegende Erfindung offenbart eine Epikard-Elektrode, also eine an der Außenseite des Herzmuskels anzubringende Elektrode, für ein Herzstimulationsgerät, wie etwa einen Defibrillator oder einen Herzschrittmacher.

Das Zuführen elektrischer Signale als Stimulationssignale, um bspw. den Herzrhythmus wieder herzustellen oder den Rhythmus des Herzschlages stabil zu halten, ist eine seit langem angewandte medizinische Therapie. Hiezu werden Herzschrittmacher, aber auch implantierbare Defibrillatoren in den Brustkorb eines Patienten eingesetzt, die mittels einer internen Energiequelle, bspw. einer Batterie, bei Bedarf die elektrischen Signale erzeugen. Diese werden dann über eine Elektrodenleitung, an deren dem Herzschrittmacher abgewandten Ende (man spricht in diesem Zusammenhang auch vom distalen Ende der Elektrodenleitung) eine Elektrode angeordnet ist, einer bestimmten, von der medizinischen Anwendung abhängigen Stelle des Herzens zugeführt.

Die Elektrode kann je nach Therapiezweck eine unipolare Elektrode oder eine bipolare Elektrode sein. Unter einer unipolaren Elektrode versteht man eine Elektrode mit einem einzigen Pol, dem sog. differenten Pol, der die Stimulationspulse aussendet. Der unipolaren Elektrode ist eine Referenzelektrode mit sog. indifferentem Pol zugeordnet. Die Referenzelektrode, die auch als indifferente Elektrode bezeichnet wird, wird häufig von einer an der Außenhaut des Herzmuskels (Epikard) angeordneten Patch-Elektrode oder vom Gehäuse des Stimulationsgerätes gebildet. An der Außenseite des Herzmuskels angebrachte Elektroden werden auch Epikard-Elektroden oder epikardiale Elektroden genannt.

Eine bipolare Elektrode weist im Gegensatz zur unipolaren Elektrode sowohl einen differenten als auch einen indifferenten Pol auf, so dass keine weitere Elektrode benötigt wird.

Zur Stimulation des Apex, also der Herzspitze, die bspw. zum Stabilisieren des Herzrhythmus erfolgt, wird im Stand der Technik eine Elektrode im Inneren des Herzens im Bereich des Apex fixiert. Dabei ist ein Durchführen der Elektrodenleitung durch die Herzkammern bzw. durch den Sinus Coronarius nötig. Eine in der Nähe des Apex der rechten Herzkammer platzierte Elektrode ist bspw. in US 5,683,447 gezeigt. Das Platzieren der Elektrode durch die Herzkammern oder den Sinus Coronarius ist jedoch nicht ungefährlich. Das Dokument US4142530 offenbart eine Epikard-Elektrode mit ausfahrbaren Krallen zur Fixierung der Elektrode im Herzgewebe. Es ist daher eine Aufgabe der Erfindung, Einführbesteck zur Verfügung zu stellen, das das Risiko für den Patienten beim Platzieren der Elektrode vermindert. Diese Aufgabe wird durch ein Einführbesteck nach Anspruch 1 gelöst. Die abhängigen Ansprüche definieren vorteilhafte Ausgestaltungen des Einführbestecks.

Eine Epikard-Elektrode, die sich insbesondere zur Verwendung mit einem Herzstimulationsgerät eignet, umfasst einen Elektrodenkörper, der eine zur Anlage an das Herzgewebe und zur Stimulation einer Partie des Herzens, d. h. eines Teilbereiches des Herzens, ausgebildete Stimulationsfläche und mindestens ein Fixierelement zum Fixieren der Stimulationsfläche am Herzgewebe aufweist. Das mindestens eine Fixierelement ist zum Eingriff in das Herzgewebe ausgestaltet.

Die Epikard-Elektrode kann an der Außenseite und insbesondere an der Außenhaut des Herzmuskels (Epikard) befestigt werden, ohne wie eine Patch-Elektrode mit dem Herzmuskel vernäht zu werden. Es muss lediglich das Fixierelement in Eingriff mit dem Herzgewebe gebracht werden. Die Elektrode erleichtert den Zugang zum Herzen und ermöglicht es insbesondere, die Elektrode in einem minimalinvasiven Eingriff anzubringen.

Darüber hinaus ermöglicht die Ausgestaltung der Stimulationsfläche derart, dass im Gegensatz zu einer Patch-Elektrode nur eine Partie, also ein Teilbereich des Herzens stimuliert wird, die gezielte Stimulation bestimmter Herzbereiche. So kann die Elektrode dazu genutzt werden, bspw. den Apex des Herzens von der Außenseite des Herzens aus zu stimulieren. Ein Platzieren der Elektrode durch die Herzkammern oder den Sinus Coronarius braucht in diesem Fall nicht zu erfolgen.

Die Möglichkeit, den Apex durch Platzieren der Elektrode zu stimulieren, ohne dass die Elektrodenleitung durch die Herzkammern oder den Sinus Coronalis geführt zu werden braucht, verringert im Vergleich zum Stand der Technik das Risiko, dem der Patient beim Platzieren der Elektrode ausgesetzt ist.

Die Elektrode erleichtert insgesamt also den insbesondere für die Stimulation des Herzrhythmus und/oder zum Abfühlen von Herzsignalen bedeutenden Zugang zum Apex des linken Herzens.

Das mindestens eine Fixierelement kann, um das Fixieren der Stimulationsfläche ohne Vernähen zu ermöglichen, insbesondere in Form einer in das Herzgewebe einbringbaren Kralle ausgestaltet sein.

In einer vorteilhaften Weiterbildung ist die Kralle derart beweglich ausgestaltet, dass sie zum Fixieren des Elektrodenkörpers am Herzgewebe aus einem in den Elektrodenköper eingezogenen Zustand zum Herstellen des Eingriffs mit dem Herzgewebe auszufahren ist. Die Kralle kann dann während des Einführens der Elektrode in den Körper in den Elektrodenkörper eingezogen sein, um das Einführen nicht zu behindern. Erst dann, wenn die Elektrode ihre Zielposition im Körper erreicht hat, wird die Kralle ausgefahren um die Stimulationsfläche zu fixieren. Besonders vorteilhaft ist diese Ausgestaltung, wenn die Kralle in der in den Elektrodenkörper eingezogenen Position vollständig im Inneren des Elektrodenkörpers angeordnet ist.

In einer weiteren Ausgestaltung der Elektrode umfasst der Elektrodenkörper zur Aufnahme der Kralle einen Kanal mit einem langgestreckten, zur Stimulationsfläche parallel verlaufenden Kanalabschnitt und einem derart gekrümmten Kanalabschnitt, dass der Kanal eine Austrittsöffnung in der Stimulationsfläche aufweist. Außerdem ist mindestens ein Teil der Kralle derart flexibel ausgebildet und im Kanal angeordnet, dass er sich im eingezogenen Zustand der Kralle im Wesentlichen langgestreckt im langgestreckten Kanalabschnitt und im ausgefahrenen Zustand im gekrümmten Kanalabschnitt befindet. Beim Ausfahren der Kralle tritt der flexible Teil der Kralle aus dem langgestreckten Kanalabschnitt in den gekrümmten Kanalabschnitt ein. In dieser Ausgestaltung verläut mindestens ein Teil der Kralle im eingezogenen Zustand parallel zur Stimulationsfläche. Der parallele Verlauf des Krallenteils im eingezogenen Zustand ermöglicht es, die Kralle vollständig in den Elektrodenkörper einzuziehen und gleichzeitig die Bauhöhe des Elektrodenkörpers relativ flach zu halten. Dies ist insbesondere von Bedeutung, wenn die Abmessungen der Elektrode gering gehalten werden sollen, um das Implantieren in einem minimalinvasiven Eingriff zu erleichtern.

Die Kralle kann insbesondere auch einen zum Eingriff in das Herzgewebe vorgesehenen steifen Krallenabschnitt aufweisen, der bogenförmig ausgebildet ist und der sich im eingezogenen Zustand im gekrümmten Kanalabschnitt befindet. Die Steife des gekrümmten Krallenabschnittes wirkt einem Verbiegen der Kralle beim Herstellen des Eingriffs mit dem Herzgewebe entgegen.

Um eine besonders sichere Befestigung der Stimulationsfläche am Herzgewebe zu gewährleisten, umfasst die Elektrode vorteilhafterweise mehrere Krallen. Insbesondere können zwei einander gegenüberliegende Krallenpaare vorhanden sein.

Um das Implantieren in einem minimalinvasiven Eingriff zu vereinfachen, ist in einer vorteilhaften Ausgestaltung der Elektrode jedem Fixierelement ein Eingriffselement zugeordnet, das einen lösbaren Eingriff eines zum Betätigen des Fixierelementes ausgestalteten Betätigungselementes ermöglicht. Insbesondere kann ein Eingriffselement, bspw. eine Öse, gleichzeitig mehreren Fixierelementen, d. h. bspw. mehreren Krallen, zugeordnet sein. Alternativ kann jedes Fixierelement jedoch auch sein eignes Eingriffselement besitzen. Die Elektrode kann dann mit dem Betätigungselement im Eingriff in das Eingriffselement am Herzen positioniert werden. Anschließend wird das Fixierelement mit Hilfe des Betätigungselementes in Eingriff mit dem Herzgewebe gebracht. Nachdem der Eingriff mit dem Herzgewebe hergestellt ist, wird er Eingriff des Betätigungselementes in das Eingriffselement gelöst. Das Betätigungselement kann dann entfernt werden.

In einer Weiterbildung dieser Ausgestaltung ermöglicht das Eingriffselement das Ausfahren einer Kralle durch im Wesentlichen paralleles Verschieben des Betätigungselementes relativ zur Stimulationsfläche, insbesondere dann, wenn der Elektrodenkörper einen langgestreckten Kanalabschnitt zur Aufnahme der Kralle aufweist.

In einer weiteren Ausgestaltung der Epikard-Elektrode weist diese an der Stimulationsfläche einen differenten Pol auf. Der differente Pol kann mit einem Reservoir eines entzündungshemmenden Wirkstoffes, bspw. eines Steroids, ausgestattet sein, um das Entstehen von Entzündungen durch das Eingreifen des Fixierelementes in das Herzgewebe zu hemmen. Mittels des differenten Pols können der zu stimulierenden Partie des Herzens die geeigneten Stimulationssignale zugeführt werden. Außerdem ermöglicht der differente Pol auch das Abfühlen elektrischer Signale des Herzens, die bspw. zum Auslösen oder Triggern der Stimulation herangezogen werden können. Zusätzlich zu dem Fixierelement kann die Stimulationsfläche noch ein medizinisches Gewebe, bspw. Dacron, zum Festwachsen mit dem Herzgewebe umfassen. Dies ermöglicht ein besonders sicheres Fixieren der Stimulationsfläche am Herzgewebe. Erfindungsgemäß wird auch ein Einführbesteck zum minimalinvasiven Einführen einer Elektrode in den Körper zur Verfügung gestellt, das einen Betätigungsmechanismus mit einem Betätigungselement zum Betätigen des mindestens einen Fixierelementes derart, dass der Eingriff des Fixierelementes mit dem Herzgewebe hergestellt wird, aufweist. Das Einführbesteck stellt damit ein Applikationswerkzeug dar, das außer zum Platzieren der Elektrode auch zum Herstellen des Eingriffs verwendet werden kann. Der Betätigungsmechanismus kann insbesondere derart ausgestaltet sein, dass er ein Ausfahren mindestens einer Kralle aus dem Elektrodenkörper ermöglicht.

Zur Verwendung für implantierbaren Elektroden, bei denen als Fixierelement eine flexible Kralle Verwendung findet, die sich im eingezogenen Zustand zumindest teilweise in einem zur Stimulationsfläche parallel verlaufenden Kanalabschnitt befindet, ist der Betätigungsmechanismus in einer Ausgestaltung des Einführbestecks derart ausgebildet, dass er ein im Wesentlichen paralleles Verschieben des Betätigungselementes relativ zur Stimulationsfläche ermöglicht. Das Verschieben des Betätigungselementes führt dann zu einem ebenfalls relativ zur Stimulationsfläche parallelen Verschieben des mit der Kralle verbundenen Eingriffselementes, was wiederum zu einem Ausfahren der Kralle aus dem Elektrodenkörper führt.

Um ein sicheres Halten der Elektrode während des Positionierens zu ermöglichen, kann das Einführbesteck außerdem mit Halteklammern zum Halten der implantierbaren Elektrode beim Einführen der implantierbaren Elektrode in den Körper ausgestattet sein.

Weitere Merkmale, Eigenschaften und Vorteile der Erfindung ergeben sich aus der nachfolgenden Beschreibung von Ausführungsbeispielen unter Bezugnahme auf die beiliegenden Figuren.
- Figur 1: zeigt in Aufsicht schematisch ein erstes Ausführungsbeispiel für die erfindungsgemäße Epikard-Elektrode.
- Figur 2: zeigt schematisch das erste Ausführungsbeispiel für die erfindungsgemäße Epikard-Elektrode in einem vertikalen Schnitt.
- Figur 3: zeigt schematisch ein erstes Ausführungsbeispiel für das Einführbesteck zum Einführen der erfindungsgemäßen Epikard-Elektrode.
- Figur 4: zeigt die Oberseite eines zweiten Ausführungsbeispiels für die erfindungsgemäße Epikard-Elektrode.
- Figur 5: zeigt die Unterseite des zweiten Ausführungsbeispiels für die erfindungsgemäße Epikard-Elektrode.
- Figuren 6 u. 7: zeigen Details der Unterseite des zweiten Ausführungsbeispiels für die erfindungsgemäße Epikard-Elektrode.
- Figur 8: zeigt in einer Gesamtansicht ein zweites Ausführungsbeispiel für das Einführbesteck für die erfindungsgemäße Epikard-Elektrode.
- Figuren 9 - 11: zeigen das zweite Ausführungsbeispiel für das erfindungsgemäße Einführbesteck in verschiedenen Stellungen.
- Figuren 12 - 16: zeigen das zweite Ausführungsbeispiel für das erfindungsgemäße Einführbesteck mit daran angeordneter Epikard-Elektrode in verschiedenen Stellungen.

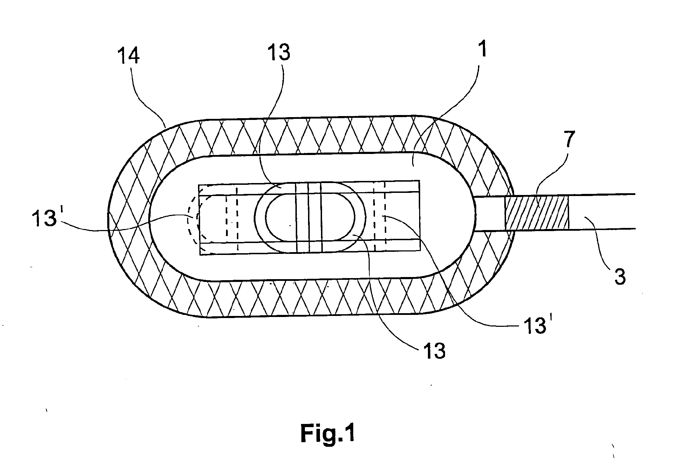

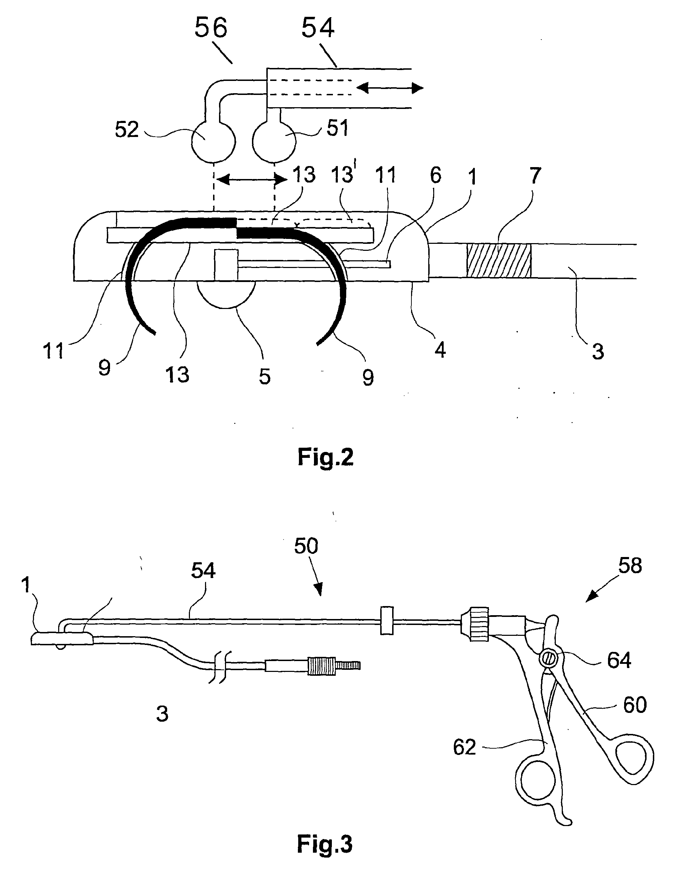

Die Figuren 1 und 2 zeigen in einer schematischen Darstellung eine bipolare Epikard-Elektrode für ein Herzstimulationsgerät als ein erstes Ausführungsbeispiel für die Epikard-Elektrode. Fig. 1 zeigt die Elektrode in Aufsicht, während Fig. 2 die Elektrode in einem vertikalen Schnitt zeigt.

Die Elektrode umfasst einen bspw. aus Silikon bestehenden Elektrodenkörper 1, der am distalen Ende einer Elektrodenleitung 3 angeordnet ist und dessen Unterseite die Stimulationsfläche 4 der Epikard-Elektrode mit einem differenten Pol 5 bildet. Über den differenten Pol 5 werden beim Stimulationsbetrieb des Stimulationsgeräts (nicht dargestellt) die Stimulationssignale an das Herz abgegeben. Zu diesem Zweck ist der differente Pol 5 über eine durch die Elektrodenleitung 3 verlaufende elektrische Leitung 6 mit dem Stimulationsgerät verbunden. Der indifferente Pol 7 ist im gezeigten Ausführungsbeispiel als ringförmige Elektrodenfläche an der Elektrodenleitung 3 angeordnet.

Zwar ist die in den Figuren 1 und 2 dargestellte Elektrode als bipolare Elektrode ausgebildet, sie kann jedoch auch als unipolare Elektrode ausgebildet sein. In diesem Fall ist kein indifferenter Pol 7 an der Elektrodenleitung vorhanden. Stattdessen dient dann z. B. das Gehäuse des Stimulationsgerätes oder eine Patch-Elektrode als indifferenter Pol.

Als Fixierelemente umfasst die Epikard-Elektrode vier Krallen 9 (von denen in Fig. 2 zwei zu erkennen sind), die einander gegenüberliegend paarweise in Kanälen 11 des Elektrodenkörpers 1 angeordnet sind. Die Krallen 9 lassen sich entlang dieser Kanäle 11 zwischen zwei Endstellungen verschieben, wobei die Krallen in der einen Endstellung, die in Fig. 2 dargestellt ist, aus dem Elektrodenkörper ausgefahren sind, so dass ihre Krallenspitzen über die Stimulationsfläche 4 vorstehen. Am den Krallenspitzen entgegengesetzten Ende der Krallen 9 sind Ösen 13 angeordnet, die sich in parallel zur Stimulationsfläche verlaufenden Abschnitten der Kanäle befinden und den Eingriff von Kugeln 51, 52 eines Einführbestecks 50, die als Betätigungselemente zum Betätigen der Krallen dienen, ermöglichen. Jedem Krallenpaar ist dabei eine gemeinsame Öse zugeordnet.

Durch gegeneinander Verschieben der Kugeln 51, 52 parallel zur Stimulationsfläche 4 lassen sich die Krallen 9 ein- und ausfahren. Insbesondere lassen sie sich aus einem Zustand, in dem sie sich vollständig innerhalb der Kanäle 11 befinden, in den ausgefahrenen Zustand bringen. Der Zustand, in dem die Krallen vollständig im Inneren der Kanäle angeordnet sind, ist durch die entsprechende, gestrichelt dargestellte Endposition der Ösen 13' angedeutet. Wenn die Stimulationsfläche 4 am Herzmuskel anliegt, dringen die Krallen 9 beim Ausfahren in das Herzgewebe ein und verankern so die Elektrode.

Als optionale Ausstattung, die eine zusätzliche Fixierung der Epikard-Elektrode am Herzgewebe ermöglicht, ist an der Unterseite der Stimulationsfläche 4 ein medizinisches Dacron-Gewebe 14 angeordnet (in Fig. 2 nicht dargestellt), das ein Festwachsen der Elektrode am Herzgewebe ermöglicht. Außerdem kann der differente Pol 5 in einer weiteren optionalen Ausgestaltung mit einem Steroid-Reservoir ausgestattet sein. Das Steroid dient dazu, Entzündungen, die beim Einbringen der Krallen in das Herzgewebe entstehen können, zu hemmen.

Zwar umfasst die Elektrode im beschriebenen Ausführungsbeispiel als Fixierelemente vier Krallen 9, die einander gegenüberliegend paarweise im Elektrodenkörper 1 angeordnet sind, jedoch kann jede beliebige Anzahl Fixierelemente zur Anwendung kommen. Die Zahl der Fixierelemente kann dabei bspw. in Abhängigkeit von der Größe der Stimulationsfläche oder vom Grad der gewünschten Fixierung gewählt werden. Figur 3 zeigt schematisch ein Ausführungsbeispiel für das Einführbesteck 50 zum Einführen der erfindungsgemäßen Epikard-Elektrode in den Körper. Eine vergrößerte Darstellung des distalen Endes des Einführbestecks ist in Fig. 2 zu erkennen. Das Einführbesteck 50 umfasst eine hohle Röhre 54, an deren distalem Ende eine zum Eingriff in die Ösen 13 der Krallen 9 ausgestaltete Kugel 51 fixiert ist. Im Inneren der hohlen Röhre 54 ist eine Stange 56 angeordnet, die sich relativ zur hohlen Röhre 54 in Längsrichtung verschieben lässt. Am Ende der Stange 56 befindet sich eine zweite zum Eingriff in die Ösen 13 der Krallen ausgestaltete Kugel 52. Durch Verschieben der Stange 56 gegen die Röhre 54 lassen sich die Kugeln 51 und 52 voneinander weg und aufeinander zu bewegen. Sind die Kugeln 51, 52 dabei in die Ösen 13 der Krallen 9 eingeloggt, so führt das Bewegen der Kugeln 51, 52 aufeinander zu zu einem Ausfahren der Krallen 9 aus den Kanälen 11 und das Bewegen der Kugeln 51, 52 voneinander weg zu einem Einziehen der Krallen 9 in die Kanäle 11.

Am proximalen Ende des Einführbestecks 50 ist ein Griff 58 mit zwei Griffteilen 60, 62 angeordnet. Die beiden Griffteile 60, 62 sind über ein Gelenk 64 derart schwenkbar zueinander angeordnet und mit der Stange 56 bzw. der Röhre 54 verbunden, dass sich die Kugeln 51, 52 aufeinander zu bewegen, wenn die beiden Griffteile 60, 62 aufeinander zu verschwenkt werden, und sich die beiden Kugeln 51, 52 voneinander weg bewegen, wenn die beiden Griffteile 60, 62 voneinander weg verschwenkt werden. Die Kugeln 51, 52, die Röhre 54, die Stange 56 sowie der Griff 58 bilden zusammen einen Betätigungsmechanismus zum Ein- und Ausfahren der Krallen 9.

Außer zum Betätigen der Krallen 9 dient das Einführbesteck 50 auch zum Platzieren der Elektrode, die dabei bspw. mittels der Kugeln 51, 52 am Einführbesteck 50 gehalten wird. Sobald die Elektrode an der richtigen Stelle des Herzens platziert ist, kann sie mit Hilfe des Betätigungsmechanismus befestigt werden. Nach dem Ausfahren der Krallen 9 wird das Einführbesteck 50 wieder entfernt.

Um das Halten der Elektrode beim Platzieren und das Entfernen des Einführbestecks 50 nach dem Fixieren der Elektrode zu ermöglichen, kann die Oberseite des Elektrodenkörpers 1 zwei sich in Verschieberichtung der Ösen 13 erstreckende Schlitze oder Langlöcher aufweisen, die dort, wo sich die Ösen 13 im ausgefahrenen Zustand der Krallen 9 befinden, derart verbreitert sind, dass die Kugeln 51, 52 nur an dieser Stelle, also im ausgefahrenen Zustand der Krallen, durch die Schlitze bzw. Langlöcher hindurchtreten können. Vor dem Einsetzen der Elektrode werden die Kugeln 51, 52 bei ausgefahrenen Krallen in die Ösen 13 eingeführt, und anschließend werden die Krallen durch Betätigen des Betätigungsmechanismus eingezogen. Dabei gelangen die Kugeln 51, 52 in diejenigen Abschnitte der Schlitze bzw. Langlöcher, die einen Durchtritt der Kugeln 51, 52 nicht erlauben. Erst nach dem Ausfahren der Krallen, also nach dem erfolgten Fixieren der Elektrode, lassen sich die Kugeln 51, 52 aus den Ösen 13 herausziehen.

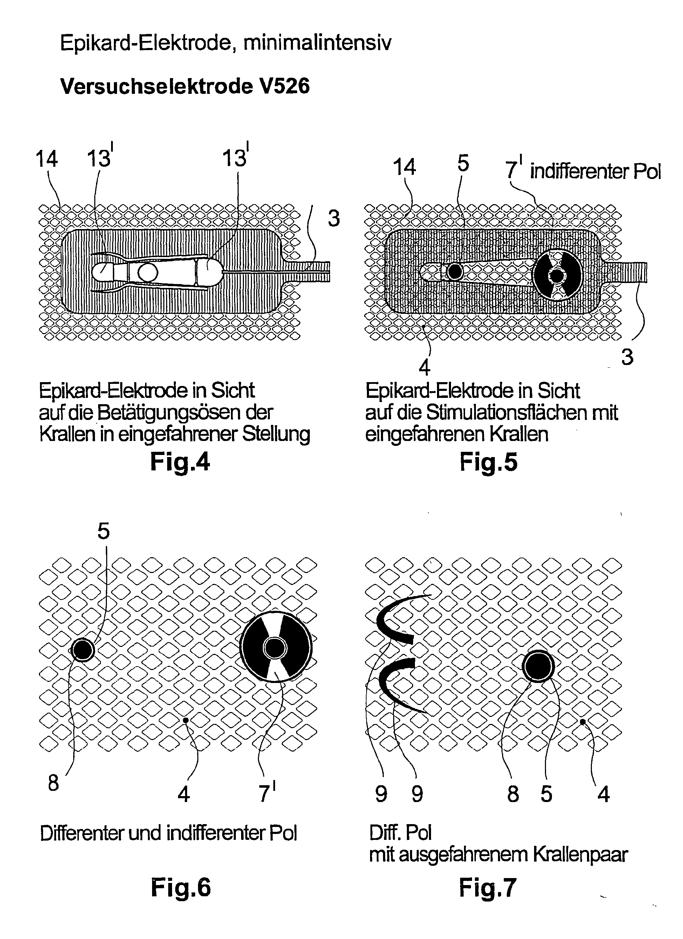

Die Figuren 4 bis 7 zeigen ein zweites Ausführungsbeispiel für die Epikard-Elektrode, wobei Figur 4 eine Ansicht auf den Elektrodenkörper 1 und Figur 5 eine Ansicht auf die Stimulationsfläche 4 zeigen. Figur 6 zeigt in einer vergrößerten Darstellung einen Ausschnitt der Stimulationsfläche 4, der den differenten und den indifferenten Pol 5 bzw. 7' der Epikard-Elektrode umfasst. In Fig. 7 ist die Stimulationsfläche 4 in einer den differenten Pol 5 sowie die ausgefahrenen Krallen 9 zeigenden vergrößerten Darstellung abgebildet. Merkmale des zweiten Ausführungsbeispiels, die sich von solchen des ersten Ausführungsbeispiels nicht unterscheiden, sind mit denselben Bezugsziffern wie im ersten Ausführungsbeispiel bezeichnet.

Das zweite Ausführungsbeispiel unterscheidet sich vom ersten lediglich dadurch, dass der indifferente Pol 7' nicht als ringförmige Elektrodenfläche an der Elektrodenleitung 3 angeordnet ist, sondern ebenso wie der differente Pol 5 an der Stimulationsfläche 4. Dadurch lässt sich ein guter Kontakt zwischen dem indifferenten Pol 7' und dem Herzgewebe erzielen. Jedoch vergrößert sich durch das Anordnen der indifferenten Elektrode 7' an der Stimulationsfläche 4 deren Abmessung, da der Abstand zwischen dem indifferenten und dem differenten Pol ca. 10 mm oder mehr betragen kann. Im dargestellten Ausführungsbeispiel ist der Elektrodenkörper 1 ca. 4,6 mm hoch, ca. 10 mm breit und ca. 25 mm lang. Die Fläche des an der Stimulationsfläche angeordneten Dacron-Gewebes beträgt ca. 14 mm x 32 mm.

Die Krallen 9 können bspw. aus Draht bestehen und typischerweise einen Durchmesser von ca. 0,2 mm aufweisen. Die Kanäle für die Krallen 9 im Elektrodenkörper 1 besitzen dann typischerweise einen Durchmesser von ca. 0,4 mm. Der differente und der indifferente Pol besitzen typischerweise einen Durchmesser von ca. 1,8 mm bzw. 4,0 mm, wobei die Dicke des differenten Pols ca. 0,6 mm und die des indifferenten Pols ca. 0,3 mm beträgt. Im Zentrum des differenten Pols befindet sich ein Steroidreservoir 8 mit einem Durchmesser von ca. 1,0 mm, welches ca. 1 mg eines Steroids, bspw. Dexamethason-Phosphat, enthält.

Die mit Bezug auf das zweite Ausführungsbeispiel genannten Abmessungen lassen sich in ihrer Größenordnung außer für die Länge des Elektrodenkörpers 1 und den Abmessungen des indifferenten Pols 7' auf das erste Ausführungsbeispiel übertragen. Ebenso lässt sich das bezüglich des Krallenmaterials gesagte auf das erste Ausführungsbeispiel übertragen.

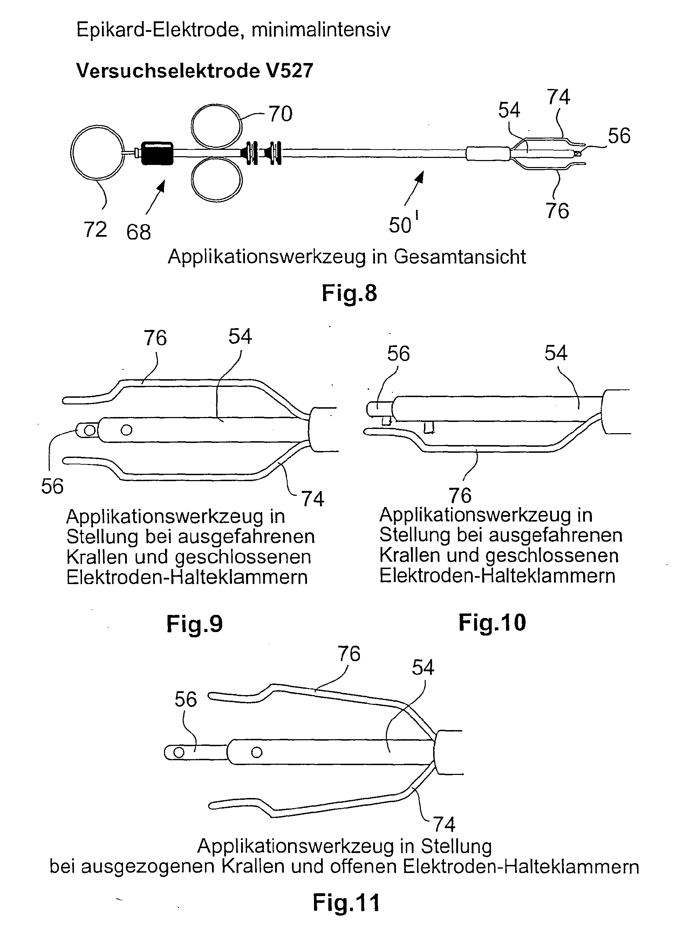

Figur 8 zeigt ein zweites Ausführungsbeispiel für das erfindungsgemäße Einführbesteck. Das Einführbesteck 50' gemäß dem zweiten Ausführungsbeispiel entspricht in seinem prinzipiellen Aufbau dem des ersten Ausführungsbeispiels. Merkmale des Einführbestecks gemäß dem zweiten Ausführungsbeispiel, die sich von denen des ersten Ausführungsbeispiels nicht unterscheiden, sind in beiden Ausführungsbeispielen mit denselben Bezugsziffern bezeichnet.

In Abweichung zum ersten Ausführungsbeispiel umfasst im zweiten Ausführungsbeispiel der Griff 68 der Betätigungsvorrichtung zum gegeneinander Verschieben der Kugeln keine zwei gegeneinander schwenkbaren Griffteile sondern zwei gegeneinander verschiebbare Griffteile 70, 72. Außerdem umfasst das Einführbesteck 50' des zweiten Ausführungsbeispiels zwei Halteklammern 74, 76 zum Halten der Elektrode während des Einführ- und des Fixiervorgangs. Die Halteklammern 74, 76 können mittels einer am Griff 68 vorgesehenen Vorrichtung wahlweise in eine geschlossene Stellung, in der sie eine Elektrode am distalen Ende des Einführbestecks halten, oder in eine offene Stellung, in der die Elektrode nicht gehalten wird, gebracht werden.

Die Figuren Fig. 9 und 10 zeigen das distale Ende des Einführbestecks 50' in der Konfiguration, die es bei ausgefahrenen Krallen der Elektrode und bei geschlossenen Halteklammern 74, 75 besitzt. Fig. 11 zeigt das distale Ende des Einführbestecks dagegen bei eingezogenen Krallen und geöffneten Halteklammern 74, 76.

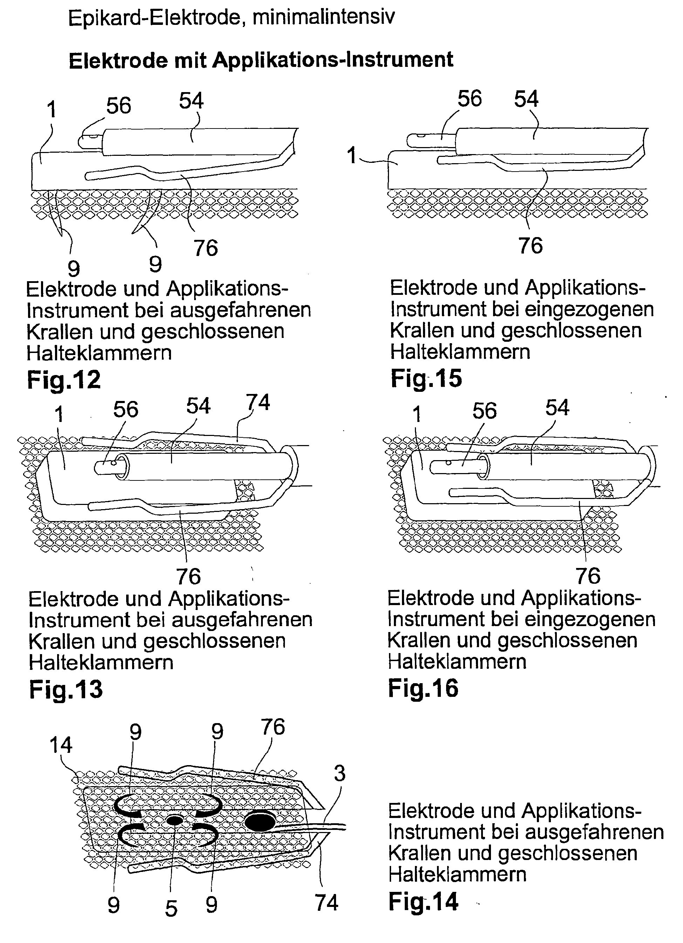

In den Figuren 12 bis 16 ist das Einführbesteck 50' gemäß dem zweiten Ausführungsbeispiel mit geschlossenen Halteklammern 74, 76 und von den Halteklammern 74, 76 gehaltener Epikard-Elektrode dargestellt. Um ein sicheres Halten der Epikard-Elektrode durch die Halteklammern 74, 76 zu unterstützen, sind in den Seitenwänden des Elektrodenkörpers 1 Nuten 16 vorhanden, in welche die Halteklammern 74, 76 im geschlossenen Zustand eingreifen. Während die Figuren 12 bis 14 die Elektrode und das distale Ende des Einführbestecks 50' unter verschiedenen Blickwinkeln bei ausgefahrenen Krallen 9 und geschlossenen Halteklammern 74, 76 zeigen, zeigen die Figuren 15 und 16 die Elektrode und das distale Ende des Einführbestecks 50' bei eingezogenen Krallen 9 und geschlossenen Halteklammern 74, 76.

Wenn die Elektrode beim Einführ- und beim Fixiervorgang von den Halteklammern 74, 76 gehalten wird, ist es ausreichend die Betätigungselemente statt als Kugeln als einfache, zum Eingriff in die Ösen 13 der Krallen 9 der Elektrode geeignete Zapfen auszubilden, da die Betätigungselemente kein Haltefunktion für die Elektrode auszuüben brauchen.

## Patentansprüche

1. Einführbesteck zum minimalinvasiven Einführen einer Epikard-Elektrode in den Körper,
wobei die Epikard-Elektrode einen Elektrodenkörper (1), der eine zur Anlage an das Herzgewebe und zur Stimulation einer Partie des Herzens ausgebildete Stimulationsfläche (4) und ein zum Eingriff in das Herzgewebe ausgestaltetes Fixierelement (9) zum Fixieren der Stimulationsfläche (4) am Herzgewebe aufweist,
mit einem Betätigungsmechanismus (51-58), der eine Stange (56) umfasst, welche im Inneren einer hohlen Röhre (54) angeordnet ist und sich relativ zur Röhre verschieben lässt,
**gekennzeichnet dadurch, dass** das Fixierelement vier Krallen umfasst, die einander gegenüberliegend paarweise im Elektrodenkörper (1) angeordnet sind und derart beweglich ausgestaltet sind, dass die gegenüberliegend paarweise angeordneten Krallen zum Herstellen eines Eingriffs mit dem Herzgewebe aus einem in den Elektrodenkörper (1) eingezogenen Zustand durch eine Aufeinander-Zubewegung von Betätigungselementen auszufahren sind, sodass die Krallenspitzen über die Stimulationsfläche (4) vorstehen, um so die Elektrode im Herzgewebe zu verankern, und dass der Betätigungsmechanismus (51 - 58) mit vier aufeinander zubeweglichen Betätigungselementen (51, 52) zum Betätigen der vier gegenüberliegend paarweise angeordneten Krallen (9) derart ausgestattet ist, dass der Eingriff der vier gegenüberliegend paarweise angeordneten Krallen (9) in das Herzgewebe erfolgt.

2. Einführbesteck nach Anspruch 1 zum Einführen einer Epikard-Elektrode, bei welchem der Betätigungsmechanismus (51 - 58) derart ausgestaltet ist, dass er ein Ausfahren der vier gegenüberliegend paarweise angeordneten Krallen (9) aus dem Elektrodenkörper (1) ermöglicht.

3. Einführbesteck nach Anspruch 2 zum Einführen einer Epikard-Elektrode, bei welchem der Betätigungsmechanismus (51 - 58) zum im Wesentlichen parallelen Verschieben der Betätigungselemente (51, 52) relativ zur Stimulationsfläche (4) ausgebildet ist.

4. Einführbesteck nach einem der Ansprüche 1 bis 3, bei dem Halteklammern (74, 76) zum Halten der Epikard-Elektrode beim Einführen in den Körper vorhanden sind.

5. Einführbesteck nach einem der vorhergehenden Ansprüche, **dadurch gekennzeichnet, dass** jedem Krallenpaar (9) ein Eingriffselement (13) in Form einer Öse zugeordnet ist, das einen lösbaren Eingriff der zum Betätigen des Krallenpaars (9) ausgestalteten Betätigungselemente (51, 52) ermöglicht.

6. Einführbesteck nach einem der vorhergehenden Ansprüche, **dadurch gekennzeichnet, dass** sich die Eingriffselemente (13) zum Ausfahren der Krallenpaare (9) durch vier Betätigungselemente (51, 52) im Wesentlichen parallel zur Stimulationsfläche (4) verschieben lassen.

## Claims

1. An insertion device for the minimally invasive insertion of an epicardial electrode into the body,
wherein the epicardial electrode comprises an electrode body (1), which has a stimulation surface (4) designed to be placed against the cardiac tissue and stimulate a section of the heart, and a fixing element (9) designed to engage into the cardiac tissue for fixing the stimulation surface (4) on the cardiac tissue, comprising an actuating mechanism (51-58), which has a rod (56), which is arranged in the interior of a hollow tube (54) and can be displaced relative to the tube,
**characterized in that**
the fixing element comprises four claws, which are arranged opposite one another in pairs in the electrode body (1) and are designed to move such that the claws, which are arranged opposite one another in pairs, are intended to be extended from a state in which said claws are retracted into the electrode body (1) by moving actuating elements toward one another in order to establish an engagement with the cardiac tissue such that the claw tips extend past the stimulation surface (4) in order to thereby anchor the electrodes in the cardiac tissue, and the actuating mechanism (51-58) has four actuating elements (51, 52), which can be moved toward one another, for actuating the four claws (9), which are arranged opposite one another in pairs, such that the four claws (9), which are arranged opposite one another in pairs, engage into the cardiac tissue.

2. The insertion device according to claim 1 for inserting an epicardial electrode, in which the actuating mechanism (51 - 58) is designed such that said actuating mechanism enables the four claws (9), which are arranged opposite one another in pairs, to be extended out of the electrode body (1).

3. The insertion device according to claim 2 for inserting an epicardial electrode, in which the actuating mechanism (51 - 58) is designed for the substantial parallel displacement of the actuating elements (51, 52) relative to the stimulation surface (4).

4. The insertion device according to any one of claims 1 to 3, in which holding clamps (74, 76) are provided for holding the epicardial electrode during insertion into the body.

5. The insertion device according to any one of the preceding claims, **characterized in that** assigned to each claw pair (9) is an engagement element (13) in the form of an eyelet, which enables a detachable engagement of the actuating elements (51, 52) designed for actuating the claw pair (9).

6. The insertion device according to any one of the preceding claims, **characterized in that** the engagement elements (13) can be displaced substantially parallel to the stimulation surface (4) in order to extend the claw pairs (9) by means of four actuating elements (51, 52).

## Revendications

1. Introducteur destiné à l'introduction mini-invasive d'une électrode épicardique dans le corps,
dans lequel l'électrode épicardique présente un corps d'électrode (1), qui présente une surface de stimulation (4) conçue pour un placement sur le tissu cardiaque et pour la stimulation d'une partie du coeur, et un élément de fixation (9) conçu pour une introduction dans le tissu cardiaque pour la fixation de la surface de stimulation (4) sur le tissu cardiaque,
avec un mécanisme d'actionnement (51 à 58), qui comprend une tige (56), laquelle est disposée à l'intérieur d'un tuyau creux (54), et qui peut se déplacer relativement par rapport au tuyau,
**caractérisé en ce que**
l'élément de fixation comprend quatre griffes, qui sont disposées par paires opposées, dans le corps d'électrode (1) et sont conçues mobiles de telle sorte que les griffes disposées par paires opposées sont à mettre dans une position de sortie pour réaliser une introduction dans le tissu cardiaque à partir d'un état en retrait dans le corps d'électrode (1) par un mouvement d'écartement des éléments d'actionnement, de sorte que les extrémités des griffes dépassent de la surface de stimulation (4) afin d'ancrer ainsi l'électrode dans le tissu cardiaque,
et que
le mécanisme d'actionnement (51 à 58), conçu avec les quatre éléments d'actionnement (51, 52) mobiles les uns sur les autres pour l'actionnement des quatre griffes (9) disposées par paires opposées, est conçu de telle sorte que l'introduction des quatre griffes (9) disposées par paires situées opposées s'effectue dans le tissu cardiaque.

2. Introducteur selon la revendication 1, pour l'introduction d'une électrode épicardique, dans lequel le mécanisme d'actionnement (51 à 58) est conçu de telle manière qu'il permet un mouvement de sortie des quatre griffes (9) disposées par paires opposées hors du corps d'électrode (1).

3. Introducteur selon la revendication 2, pour l'introduction d'une électrode épicardique, dans lequel le mécanisme d'actionnement (51 à 58) est conçu pour un déplacement dans l'ensemble parallèle des éléments d'actionnement (51, 52) relativement par rapport à la surface de stimulation (4).

4. Introducteur selon l'une des revendications 1 à 3, dans lequel des pinces de maintien (74, 76) sont présentes pour le maintien de l'électrode épicardique lors de l'introduction dans le corps.

5. Introducteur selon l'une des revendications précédentes, **caractérisé en ce qu'**un élément d'introduction (13) sous la forme d'un oeillet, qui permet une introduction rétractable des éléments d'actionnement (51, 52) conçus pour l'actionnement de la paire de griffes (9), est associé à chaque paire de griffes (9).

6. Introducteur selon l'une des revendications précédentes, **caractérisé en ce que** les éléments d'introduction (13) peuvent être déplacés par quatre éléments d'actionnement (51, 52) dans l'ensemble parallèles à la surface de stimulation (4) pour la sortie de la paire de griffes (9).
